# FASCICULE DE BREVET EUROPEEN

(11) **EP 0 801 953 B1**
(45) Date de publication et mention de la délivrance du brevet: **03.07.2002**
(21) Numéro de dépôt: 97420063.6
(22) Date de dépôt: 17.04.1997
(51) Int. Cl.: A61L 27/00, A61L 29/00, A61L 33/00, B01D 71/42, B01D 69/08, B01D 67/00, A61M 1/36

(54) **Appareil médical pour le traitement extracorporel du sang ou du plasma et procédés de fabrication de cet appareil**
Medizinische Vorrichtung zur extrakorporalen Blut-oder Blutplasmabehandlung und Herstellungsverfahren für diese Vorrichtung
Medical device for the extracorporal blood or plasma treatment and methods for the manufacture of such device

(30) Priorité: 19.04.1996 FR 9605189; 19.07.1996 FR 9609340
(43) Date de publication de la demande: 22.10.1997
(73) Titulaire: HOSPAL INDUSTRIE, F-69883 Meyzieu Cédex (FR)
(72) Inventeur: Burtin, Jacques, 69320 Feyzin (FR); Thomas, Michel, 69360 Serezin du Rhône (FR); Valette, Pierre, 69330 Meyzieu (FR)
(74) Mandataire: Lejeune, Daniel

(56) Documents cités:
- EP-A- 0 561 379
- EP-A- 0 568 451
- WO-A-96/02288
- DE-A- 2 812 174
- US-A- 4 545 910

## Description

La présente invention a pour objet un appareil médical pour le traitement extracorporel du sang ou du plasma, permettant de prévenir les conséquences indésirables de l'activation de la phase contact. La présente invention a également pour objet des procédés de fabrication d'un appareil médical pour le traitement extracorporel du sang ou du plasma permettant de prévenir les conséquences indésirables de l'activation de la phase contact connues sous le nom de réactions anaphylactoïdes.

Les réactions anaphylactoïdes se produisent parfois chez des patients soumis à divers traitements du sang par circulation extracorporelle. Ces réactions se manifestent quelques minutes après le début du traitement par divers symptômes, parmi lesquels la sensation de chaleur généralisée, l'engourdissement des doigts, des lèvres ou de la langue, le halètement, la nausée, l'oedème laryngé.

Des réactions anaphylactoïdes ont été observées notamment chez les insuffisants rénaux traités par hémodialyse, hémofiltration ou hémodiafiltration au moyen d'un échangeur à membrane. Elles ont été observées avec des échangeurs ayant des membranes de différentes compositions chimiques, tantôt lors d'une utilisation unique, tantôt après plusieurs utilisations lorsque les échangeurs, au lieu d'être jetés après un usage unique, sont réutilisés de multiples fois et sont recyclés après chaque usage. Comme exemple d'échangeurs dont une première utilisation s'est accompagnée d'une réaction indésirable, on peut citer les dialyseurs ayant une membrane de polyméthylméthacrylate et de polyacrylonitrile. Des réactions associées à la réutilisation des dialyseurs à membrane d'acétate de cellulose et de polysulfone ont été également bien documentées (voir D.A.P. et al "Anaphylactoid reactions associated with reuse of hollow-fiber hemodialyzers and ACE inhibitors" Kidney International 42, 1232-1237 (1992).

Les réactions anaphylactoïdes sont imputées à une concentration excessive, dans le sang ou le plasma, d'une substance peptidique, la bradykinine. Une explication, qui a été proposée pour rendre compte de ces réactions indésirables chez certains patients soumis à un traitement de sang par circulation extracorporelle, part de ce que le sang, lorsqu'il entre en contact avec la surface négativement chargée de certains appareils de traitement de sang, est le siège d'un phénomène biologique, appelé activation de la phase contact, aboutissant à la génération de substances actives, la kallicréine et le facteur XIIa à partir de substances inactives, prékallicréine et facteur XII, la kallicréine ayant un effet catalytique sur la production du facteur XIIa et inversement. Or, la bradykinine résulte de la transformation opérée par la kallicréine - engendrée lors de l'activation de la phase contact - d'une protéine du plasma, le kininogène de haut poids moléculaire.

Il faut souligner que l'activation de la phase contact semble se produire notamment quand un dispositif de traitement du sang présentant une surface chargée négativement est utilisé, sans que, en l'absence de facteurs perturbateurs, les patients en éprouvent le moindre inconfort. Des complications surviennent parfois en présence de facteurs perturbateurs comme, par exemple :
- la présence, dans le sang à traiter, de médicaments pour combattre l'hypertension artérielle par inhibition du mécanisme naturel de la vasoconstriction, génériquement désignés par le vocable d'inhibiteurs d'enzyme de conversion ou IEC. Ces IEC sont également utilisés pour d'autres applications thérapeutiques, notamment pour traiter certaines formes d'insuffisance cardiaque. Or, les IEC ont également pour effet d'empêcher la dégradation de la bradykinine.
- la dilution du sang entrant dans le dialyseur rempli de solution saline et l'abaissement concomitant du pH du sang.

Afin d'éviter la génération de bradykinine à un niveau supérieur à 4000 pg/ml, la demande de brevet européen n° 0561379 recommande de ne mettre en contact avec le sang ou le plasma que des membranes semi-perméables présentant une densité de charges limitée en surface, à savoir une charge électrique en surface supérieure ou égale à - 30 µeq/g de polymère (soit - 30 meq/kg de polymère).

Il semble cependant que, au sens de la demande de brevet européen n° 0561379, la charge électrique en surface doive être interprétée comme la capacité ionique globale. Dès lors, cette demande de brevet européen ne concerne que des membranes semi-perméables ayant une densité de charges limitée, mais pas des membranes semi-perméables ayant une densité de charges négatives élevée comme, par exemple, la membrane fabriquée à partir d'un copolymère d'acrylonitrile et de méthallylsulfonate de sodium connue sous le nom commercial AN69.

Par ailleurs, pour obtenir des membranes ayant une charge électrique de surface limitée, c'est-à-dire aussi une faible capacité ionique globale, cette demande de brevet propose de traiter le polymère de façon à en abaisser la capacité ionique dans la masse. Or, il présente un intérêt de ne pas modifier la capacité ionique des membranes car cette capacité favorise l'adsorption de certaines protéines indésirables telles que la β2-microglobuline, les médiateurs de l'inflammation, les lipides, les facteurs du complément. Cela est particulièrement vrai pour les membranes ayant une structure homogène, symétrique.

On connaît d'autre part des composés chimiques ayant une activité antiprotéase importante qui se caractérise par une action inhibitrice dans le système de la coagulation, dans le système du complément et dans le système kallicréines-kinines (inhibition des kallicréines plasmatiques et tissulaires). A ce titre, il faut citer le nafamostat mésilate (nom chimique : 6-amideno-2-naphtyl p-guanidinobenzoate diméthane sulfonate) commercialisé par la société TORII PHARMACEUTICAL.

Le nafamostat mésilate est essentiellement utilisé en tant qu'anti-coagulant dans les circulations extracorporelles en chirurgie cardiaque, en plasmaphérèse et en aphérèse anti LDL et en hémodialyse. Le protocole d'utilisation généralement utilisé et le plus souvent préconisé est le suivant :
(a) addition de 20 mg de nafamostat mésilate dans 500 ml de solution saline pour le rinçage de l'appareil médical avant utilisation;
(b) perfusion en continu de nafamostat mésilate à raison de 20 à 50 mg/heure.

Un tel protocole implique l'utilisation de quantités importantes de nafamostat mésilate, généralement plus de 100 mg de nafamostat mésilate par séance d'hémodialyse. Un inconvénient de ce composé est d'être beaucoup plus onéreux que les anticoagulants classiques comme l'héparine.

En outre, des études ont montré que les résultats en matière d'anticoagulation ne sont pas satisfaisants quand le nafamostat mésilate est utilisé en hémodialyse avec les membranes chargées négativement comme des membranes en polyméthacrylate de méthyle ou en polyacrylonitrile, en particulier la membrane de dénomination commerciale AN69 de la société HOSPAL.

Des études ont montré que ces résultats non satisfaisants coïncident avec la forte adsorption du nafamostat mésilate sur ces membranes et que cette adsorption conduit à une diminution de l'activité anticoagulante de ce composé. Ceci ressort notamment des publications qui suivent :
- Y. Tsubakihara et al. "Anticoagulant activity of FUT-175 in polyacrylonitrile membrane dialysers". [Abstract n° 119 of the 9th annual meeting of the International Society of Blood Purification], Blood Purification 9, 1, 51-52 (1991).
- O. Inagaki et al. "Study of the binding capacity of nafamostat mesilate (NM) to dialysis membranes". Artif. Org. 15, 4, 287 (1991).
- O. Inagaki et al. "Adsorption of nafamostat mesilate by hemodialysis membranes". Artif. Org. 16, 6, 553-558 (1992).

Afin de préparer un plasma ayant une faible teneur en bradykinine, la demande de brevet japonais publiée le 13 Décembre 1994 sous le n° 6-340536, préconise un appareil médical dans lequel l'élément actif en adsorption, chargé d'adsorber et d'éliminer les composés indésirables du plasma, est porteur de groupes anioniques. En outre, cet appareil médical est pourvu :
- d'un moyen pour le mélange d'un agent antiprotéase, comme le nafamostat mésilate, et du plasma avant le traitement de ce dernier ;
- d'un moyen pour la perfusion du mélange d'agent antiprotéase et de plasma sur l'élément actif en adsorption ; et
- d'un moyen pour l'adsorption et l'élimination des composés indésirables du plasma à l'aide de l'élément actif en adsorption ;

La quantité de solution d'antiprotéase admise.dans cet appareil médical est comprise entre 10 µmoles et 60 µmoles par heure (soit environ 5 à 30 mg/heure).

Selon cette demande de brevet, il est préférable de prétraiter, avant utilisation, l'élément actif en adsorption avec une solution de prétraitement contenant un agent antiprotéase. En outre, afin d'éviter que l'agent antiprotéase ne soit endommagé par le traitement de stérilisation, il est recommandé de prétraiter l'élément actif en adsorption après le traitement de stérilisation. Egalement, afin d'éviter que l'agent antiprotéase ne soit endommagé par les rayons ultraviolets, la chaleur ou tout autre phénomène pouvant se produire durant le stockage, on recommande de prétraiter l'élément actif en adsorption juste avant son utilisation, soit au plus 24 heures avant l'utilisation si le stockage s'effectue à une température ambiante ou au plus 72 heures avant utilisation si le stockage s'effectue à une température inférieure, comprise entre 2°C et 8°C.

Dans le cas où le plasma est traité de façon intermittente par l'élément actif en adsorption, il est conseillé, dans cette demande de brevet, de prétraiter cet élément actif avant le traitement de chaque fraction de plasma.

D'autre part, pour éviter d'utiliser de trop grandes quantités d'agent antiprotéase, cette demande de brevet recommande de choisir un élément actif en adsorption dont la quantité de groupes anioniques présents en surface soit limitée, c'est-à-dire soit de préférence comprise entre 0,5 µeq et 100 µeq par ml de polymère gonflé de l'élément actif en adsorption, et de préférence encore, soit comprise entre 1 µeq et 50 µeq par ml de polymère gonflé de l'élément actif en adsorption (par "polymère gonflé", on entend l'élément actif en adsorption hydraté à la teneur correspondant à l'utilisation clinique).

Un inconvénient du procédé proposé par la demande de brevet japonais n° 6-340536 est qu'il n'est pas industrialisable, sa mise en oeuvre devant être effectuée sur le lieu du traitement.

Compte tenu de ce qui précède, à cette date, il ne semble pas qu'une solution satisfaisante, à la fois sur le plan médical et sur le plan économique, ait été apportée pour empêcher l'apparition des réactions anaphylactoïdes chez certains patients soumis à un traitement du sang ou du plasma, à l'aide d'un appareil médical dans lequel une partie au moins de l'appareil venant en contact avec le sang ou le plasma présente une densité de charges électronégatives en surface.

Un but de l'invention est donc de réaliser un appareil médical à membrane semi-perméable pour le traitement extracorporel du sang ou du plasma qui présente deux caractéristiques considérées comme antinomiques jusqu'à présent, à savoir une capacité ionique globale élevée (qui tout à la fois participe à la biocompatibilité de certains matériaux et est un facteur de déclenchement de l'activation de la phase contact), et l'aptitude à prévenir les effets indésirables de l'activation de la phase contact.

En d'autres termes, un but de l'invention est un appareil médical à membrane semi-perméable pour le traitement extracorporel du sang ou du plasma, dont la membrane est apte à prévenir les effets indésirables de l'activation de la phase contact et est quasiment insensible aux agents oxydants et aux rayonnements, en particulier les rayonnements gamma, pour pouvoir être stérilisée.

Un autre but de l'invention est un appareil médical à membrane semi-perméable pour le traitement extracorporel du sang ou du plasma sensiblement stable au stockage ainsi que lors de son utilisation.

Enfin, un but de l'invention est un appareil médical à membrane semi-perméable pour le traitement extracorporel du sang qui soit industrialisable, c'est-à-dire qui soit prêt à l'emploi et qui n'exige pas de manipulation spéciale de la part de l'utilisateur de l'appareil médical aux fins de prévenir les effets indésirables de l'activation de la phase contact.

Pour atteindre ces différents buts, la présente invention propose un appareil médical pour le traitement extracorporel de sang ou de plasma comprenant une membrane semi-perméable constituée d'au moins un polymère électronégatif, de telle sorte que la surface de la membrane semi-perméable présente des charges électriques négatives, cet appareil étant caractérisé en ce que, avant la stérilisation de l'appareil et avant ou après la formation de la membrane, au moins un agent cationique et antiprotéase est incorporé à au moins une partie de la membrane, cet agent étant adsorbé massiquement ou semi-massiquement dans la membrane et/ou lié par une interaction ionique avec les sites électronégatifs de la membrane.

Par "traitement extracorporel du sang", on entend, par exemple, l'hémodialyse, l'hémofiltration ou l'hémo-diafiltration, la plasmaphérèse.

Par "membrane semi-perméable", on entend une membrane plane ou un faisceau de fibres creuses. Dès lors, l'appareil selon l'invention comprend, en général, deux compartiments séparés par la membrane semi-perméable.

Par "adsorbé massiquement ou semi-massiquement", on entend une fixation de l'agent cationique et antiprotéase par liaisons électrostatiques, notamment ioniques, dans l'épaisseur et à la surface de la membrane.

De façon surprenante, il a été trouvé qu'il est possible de prévenir les conséquences indésirables de l'activation de la phase contact, qui peuvent survenir épisodiquement lors du traitement des liquides biologiques destinés à être restitués au corps humain tels que le plasma ou le sang, avec des membranes semi-perméables et anioniques, en incorporant à ces membranes, avant stérilisation, au moins un agent cationique ayant une action antiprotéase vis-à-vis du système contact kallicréines-kinines.

En outre, l'appareil médical conforme à l'invention n'exige pas de manipulation spéciale de la part de l'utilisateur de l'appareil aux fins de prévenir les effets indésirables de l'activation de la phase contact, comme le traitement de la membrane avec un agent antiprotéase avant le traitement médical et/ou la perfusion en continu d'agent antiprotéase pendant le traitement médical.

L'agent cationique et antiprotéase préféré est le nafamostat mésilate.

Un avantage du nafamostat mésilate de première importance pour les appareils médicaux pour le traitement extracorporel du sang ou du plasma réside dans le fait que l'incorporation de faibles quantités de cet agent à une membrane semi-perméable ayant une capacité ionique globale élevée permet de prévenir les effets indésirables de l'activation de la phase contact. Ainsi, comme indiqué ci-après (dans l'exemple 5), 7 mg de nafamostat mésilate par m² de surface totale de membrane en AN69 sont suffisants, sous réserve de suivre les recommandations d'utilisation des appareils indiquées ci-après, variables selon les modalités de fabrication de l'appareil médical,

Conformément à l'invention, la membrane semi-perméable utile à la réalisation de l'appareil médical pour le traitement extracorporel du sang ou du plasma permettant de prévenir les effets indésirables de l'activation de la phase contact est constituée d'une combinaison d'au moins un polymère électronégatif et d'au moins un agent cationique et antiprotéase.

En outre, l'invention est particulièrement bien adaptée aux appareils médicaux dans lesquels la membrane semi-perméable présente une capacité ionique globale élevée.

L'invention est particulièrement bien adaptée aux appareils médicaux dans lesquels la membrane semi-perméable présente une densité de charges négatives supérieure à 100 µeq par ml de polymère gonflé (à titre de référence, le polymère électronégatif utilisé pour réaliser la membrane en AN69 présente une densité de charges négatives égale à environ 180 µeq par ml de polymère gonflé) telle que mesurée par la méthode classique d'échanges d'ions.

Avantageusement, la membrane semi-perméable est une membrane plane ou un faisceau de fibres creuses constituée d'au moins un polymère électronégatif qui peut être l'homo- ou un co-polymère de l'acrylonitrile.

A titre d'exemple de copolymères d'acrylonitrile, on peut citer :
(1) un copolymère de l'acrylonitrile et d'au moins un monomère anionique ou anionisable, renfermant, le cas échéant, des unités provenant d'au moins un autre monomère à insaturation oléfinique capable d'être copolymérisé avec l'acrylonitrile, ou
(2) un copolymère de l'acrylonitrile et d'au moins un monomère anionique ou anionisable et d'au moins un monomère non ionique et non ionisable.

Certains de ces composés macromoléculaires, ainsi que les divers monomères susceptibles d'être retenus comme matières premières et leur fabrication, sont décrits dans le brevet américain n° 4,545,910 redélivré sous le n° Re.34239.

Parmi ces composés macromoléculaires, ceux avec lesquels l'appareil médical selon l'invention est particulièrement bien adapté sont définis plus haut sous (1). En particulier, l'invention convient particulièrement bien aux composés pour lesquels le comonomère anionique ou anionisable est oléfiniquement insaturé et porteur de groupements anioniques choisis parmi les groupes sulfonate, carboxyle, phosphate, phosphonate et sulfate, et plus particulièrement encore, quand ce comonomère est le méthallylsulfonate de sodium.

Bien entendu, la nature précise du contre-ion des groupements anioniques n'est pas essentielle au bon fonctionnement de l'invention.

Parmi les monomères à insaturation oléfinique capables d'être copolymérisés avec l'acrylonitrile, on peut citer les acrylates d'alkyle et, en particulier, l'acrylate de méthyle.

L'invention a également pour objet :
- des procédés de préparation d'une membrane plane ou d'une fibre creuse, utile à la réalisation de la membrane semi-perméable de l'appareil médical pour le traitement extracorporel du sang ou du plasma;
- un procédé de fabrication de l'appareil médical pour le traitement extracorporel du sang ou du plasma.

Un premier procédé de préparation d'une membrane plane constituant la membrane semi-perméable de l'appareil médical pour le traitement extracorporel du sang ou du plasma comprend les étapes de :
- préparer une solution comprenant :
   · au moins un polymère électronégatif,
   · au moins un solvant du polymère électronégatif,
   · éventuellement, au moins un non solvant du polymère électronégatif,
- extruder cette solution au travers d'une filière plane ;
- après l'extrusion, procéder à la solidification pour former la membrane semi-perméable plane par un procédé de solidification par inversion de phase par contact avec un liquide chimiquement inerte vis-à-vis du polymère électronégatif, ce liquide renfermant au moins un agent antiprotéase et cationique, à l'état dissous ;
- éventuellement étirer la membrane semi-perméable plane obtenue, et
- laver la membrane semi-perméable obtenue.

Un deuxième procédé de préparation d'une membrane plane constituant la membrane semi-perméable de l'appareil médical pour le traitement extracorporel du sang ou du plasma comprend les étapes de :
- préparer une solution comprenant :
   · au moins un polymère électronégatif,
   · au moins un solvant du polymère électronégatif,
   · éventuellement, au moins un non solvant du polymère électronégatif,
- extruder cette solution au travers d'une filière plane ;
- après l'extrusion, procéder à la solidification pour former la membrane semi-perméable par un procédé de solidification par inversion de phase par contact avec un liquide chimiquement inerte vis-à-vis du polymère électronégatif ;
- éventuellement étirer la membrane semi-perméable plane obtenue ; et
- laver la membrane semi-perméable obtenue avec une solution de lavage renfermant au moins un agent antiprotéase et cationique, à l'état dissous.

Un troisième procédé de préparation d'une fibre creuse constituant la membrane semi-perméable de l'appareil médical pour le traitement extracorporel du sang ou du plasma comprend les étapes de :
- préparer une solution comprenant :
   · au moins un polymère électronégatif,
   · au moins un solvant du polymère électronégatif,
   · éventuellement, au moins un non solvant du polymère électronégatif,
- extruder cette solution pour former une fibre creuse et simultanément solidifier la fibre creuse obtenue par un procédé d'inversion de phase par contact partiel ou total du produit extrudé avec un liquide de centrage chimiquement inerte vis-à-vis du polymère électronégatif et renfermant au moins un agent antiprotéase et cationique à l'état dissous,
- éventuellement étirer la fibre creuse obtenue ; et
- laver la fibre creuse obtenue.

Un quatrième procédé de préparation d'une fibre creuse constituant la membrane semi-perméable de l'appareil médical pour le traitement extracorporel du sang ou du plasma comprend les étapes de :
- préparer une solution comprenant :
   · au moins un polymère électronégatif,
   · au moins un solvant du polymère électronégatif,
   · éventuellement, au moins un non solvant du polymère électronégatif,
- extruder cette solution pour former une fibre creuse et simultanément solidifier la fibre creuse obtenue par un procédé d'inversion de phase par contact partiel ou total du produit extrudé avec un liquide de centrage chimiquement inerte vis-à-vis du polymère électronégatif,
- éventuellement étirer la fibre creuse obtenue ; et
- laver la fibre creuse obtenue avec une solution de lavage renfermant au moins un agent antiprotéase et cationique à l'état dissous.

Les autres conditions opératoires classiques des quatre procédés précités pourront être trouvées dans le brevet US 4 749 619 (procédé par gélification) ou dans le brevet US 4 056 467 (procédé par coagulation).

Conformément à l'invention, un appareil médical est fabriqué avec l'une des membranes planes ou fibres creuses obtenues grâce à l'un des procédés précités. Les principales étapes de fabrication sont les suivantes :
- préparer une membrane semi-perméable de superficie déterminée à partir de la membrane plane ou de la fibre creuse, la fibre creuse étant conformée en un faisceau de fibres creuses ;
- assembler les divers composants de l'appareil médical, en particulier monter la membrane plane ou le faisceau de fibres creuses dans un boîtier et, le cas échéant, fixer des embouts au boîtier.

L'invention a également pour objet un procédé de fabrication d'un appareil médical pour le traitement extracorporel du sang ou du plasma comprenant une membrane semi-perméable délimitant deux compartiments à l'intérieur d'un boîtier, la membrane étant constituée d'au moins un polymère électronégatif et ayant une surface présentant des charges électriques négatives, ce procédé comprenant les étapes de :
- préparer une membrane plane ou une fibre creuse ;
- assembler les divers composants de l'appareil, en particulier monter une membrane semi-perméable plane ou un faisceau de fibres creuses dans un boîtier ;
- préparer une solution contenant l'agent cationique et antiprotéase à l'état dissous, le pH de cette solution étant acide ou neutre (i.e. pH inférieur ou égal à 7) ;
- avant de stériliser l'appareil médical, porter cette solution au contact d'au moins une partie de la membrane semi-perméable, puis purger l'appareil de cette solution.

De préférence, la solution comprenant l'agent cationique et antiprotéase est portée au contact d'au moins une partie de la surface de la membrane située du côté du compartiment de l'appareil destiné à recevoir le sang ou le plasma du patient.

Selon un premier mode de réalisation de l'invention, l'étape de porter la solution au contact de la membrane consiste à faire circuler la solution comprenant l'agent cationique et antiprotéase d'une extrémité à l'autre du compartiment sang, puis à inverser le sens de circulation de la solution de façon que chaque extrémité du compartiment soit en contact avec la solution contenant l'agent antiprotéase à la concentration de préparation.

Un avantage important de ce premier mode de réalisation de l'invention réside dans le fait que les deux tubulures d'accès au compartiment sang sont équivalentes en ce sens que l'une peut servir pour l'entrée du sang et l'autre pour la sortie du sang, ou inversement. En outre, toujours dans le cadre de ce premier mode de réalisation de l'invention, la quantité d'agent cationique et antiprotéase à incorporer à la membrane semi-perméable est faible. Ainsi, comme indiqué ci-après (dans l'exemple 5), 7 mg de nafamostat mésilate par m² de surface totale de membrane en AN69 suffisent pour ne pas provoquer une élévation du taux de kallicréines plasmatiques dans un plasma dilué.

Selon un second mode de réalisation de l'invention, l'étape de porter la solution comprenant l'agent cationique et antiprotéase au contact de la membrane d'une extrémité à l'autre du compartiment sang, consiste à faire circuler la solution dans un seul sens de circulation, inverse de celui prévu pour le sang ou le plasma du patient.

En pratique, pour faciliter l'utilisation de l'appareil médical fabriqué selon ce second mode de réalisation de l'invention, avant de porter une solution contenant l'agent cationique et antiprotéase au contact de la membrane, on repère l'entrée et la sortie du compartiment de l'appareil destiné à recevoir le sang ou le plasma du patient afin que l'entrée de la solution d'agent cationique et antiprotéase soit utilisée en tant que sortie sang et que la sortie de la solution d'agent cationique et antiprotéase soit utilisée en tant qu'entrée sang.

De préférence, le débit de la solution comprenant l'agent cationique et antiprotéase que l'on fait circuler dans un seul sens, inverse de celui prévu pour le sang et le plasma du patient, est faible. Ainsi, dans le cas précité du nafamostat mésilate et de la membrane en AN69, il est avantageux de limiter le débit de la solution à une valeur inférieure à 100 ml/min., de préférence encore de l'ordre de 50 ml/min. ou inférieur.

Une technique de stérilisation qui peut être employée sans dommage pour l'agent cationique et antiprotéase ni désorption de cet agent est la stérilisation par irradiation gamma.

### Exemples

### Exemple 1

Un dialyseur a été fabriqué conformément à l'invention par la société HOSPAL INDUSTRIE, Meyzieu (Rhône), France. Une fibre creuse a été fabriquée par le procédé de gélification suivant : un mélange de copolymère d'acrylonitrile et de méthallyle sulfonate de sodium (35% en masse ; 580 mEq/kg de polymère sec), de diméthyle formamide (52% en masse) et de glycérol (13% en masse) a été préparé. Ce mélange a été porté à 130°C et a été extrudé au travers d'une filière annulaire au moyen d'un dispositif couplé d'extrusion/pompage. Le fluide de centrage utilisé pour ménager la cavité interne de la fibre était de l'azote sec. Au sortir de la filière la fibre a été reçue dans un bain contenant un mélange d'eau et d'eau oxygénée à 10°C, puis a été étirée dans un bain d'eau à 95°C (longueur multipliée par quatre). Enfin la fibre a été plastifiée avec un mélange d'eau et de glycérol (60% en masse). La densité de charges électriques ou capacité ionique globale de cette fibre creuse était de 180 µeq/ml de copolymère gonflé.

Un faisceau d'environ 9000 fibres creuses a été conformé. La surface utile de la membrane à l'état humide était d'environ 1,3 m². La surface totale en contact avec le sang est de 1,43 m². Ce faisceau a été inséré dans un boîtier tubulaire en polycarbonate où il a été fixé à ses deux extrémités par un disque de colle polyuréthanne emprisonnant les fibres sur une partie de leur longueur. Les deux disques de colle délimitent entre eux un compartiment étanche, dans lequel, lors de l'utilisation du dialyseur, un liquide de dialyse est mis en circulation par deux tubulures d'entrée/sortie. Un second compartiment est délimité par l'intérieur des fibres et deux embouts, munis chacun d'une tubulure d'accès, fixés aux extrémités du boîtier. Lors de l'utilisation du dialyseur, c'est dans ce second compartiment que le sang du patient est mis en circulation.

Une solution a été préparée, constituée d'eau déminéralisée dans laquelle du nafamostat mésilate (commercialisé sous la dénomination FUTHAN par la société TORII PHARMACEUTICAL) a été dissous dans une proportion de 20 mg/litre. Le pH de cette solution est acide (i.e. égal à 4,3).

Après assemblage des différentes composantes du dialyseur (boîtier, faisceau de fibres et embouts d'extrémités) et avant stérilisation, un litre de cette solution a été mis en circulation à un débit de 300 ml/min. dans le compartiment sang du dialyseur, dans un seul sens de circulation, inverse de celui prévu pour le sang ou le plasma, puis on a purgé le dialyseur de cette solution.

Deux jours plus tard, le dialyseur a été stérilisé par irradiation gamma.

Pour mesurer l'efficacité du procédé de cette fabrication, on a fait subir au dialyseur ainsi fabriqué le test suivant :

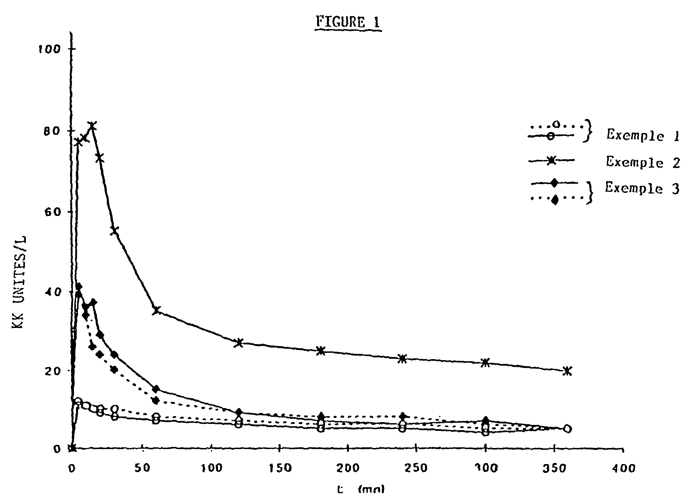

Un liquide biologique, propre à stimuler la production de kallicréines (KK) au contact de cette membrane chargée négativement en surface, a été préparé. Le liquide biologique utilisé pour l'essai était constitué de plasma humain pauvre en plaquettes, dilué à 5 %. Deux litres de ce liquide ont été mis en circulation en circuit fermé dans le dialyseur à un débit de 100 ml/mn pendant six heures : le sens de circulation de ce liquide biologique dans le compartiment sang du dialyseur était inverse de celui de la solution renfermant le nafamostat mésilate. Les kallicréines plasmatiques ont été dosées dans des échantillons de liquide prélevés à intervalle de temps (t) par un test chromogénique classique, à partir du substrat S 2302 de la société BIOGENIC. Il apparaît clairement sur la figure 1 annexée que le dialyseur fabriqué conformément à l'invention ne provoque pas l'élévation du taux de kallicréines plasmatiques dans un plasma dilué.

A cet égard, on considère dans la présente demande que, en tenant compte du test chromogénique utilisé et de sa sensibilité, il n'y a pas d'élévation significative du taux de kallicréines si sa concentration reste en deçà d'environ 20 unités par litre, et encore mieux en deçà d'environ 10 unités par litre.

### Exemple 2

Cet exemple illustre l'importance du sens de circulation des liquides biologiques lorsque l'incorporation du nafamostat mésilate a été réalisée par circulation, dans un seul sens, d'une solution renfermant cet agent dans le compartiment sang.

Pour ce faire, on a reproduit les conditions de fabrication et de test d'évaluation de l'exemple 1 en modifiant uniquement le sens de circulation du liquide biologique afin qu'il soit le même que le sens de circulation de la solution constituée d'eau déminéralisée et de nafamostat mésilate.

Il apparaît clairement sur la figure 1 annexée, une élévation du taux de kallicréines plasmatiques dans un plasma dilué.

### Exemple 3

A titre de comparaison, un test chromogénique classique réalisé dans les conditions de l'exemple 1 a été effectué avec un dialyseur de type PAN 17 DX fabriqué par la société ASAHI MEDICAL CO (faisceau de fibres creuses faites à partir d'un polyacrylonitrile ; surface utile, 1,7 m²).

Il apparaît clairement, sur la figure 1 annexée, que ce dialyseur dont la membrane présente des charges électriques négatives en surface, provoque une élévation importante du taux des kallicréines plasmatiques dans un plasma dilué.

### Exemples 4 et 5

Ces exemples illustrent divers appareils médicaux conformes à l'invention dans lesquels on a fait varier la quantité de nafamostat mésilate à incorporer dans la membrane.

Pour ce faire, on a reproduit une grande partie des conditions de fabrication et d'évaluation de l'exemple 1 en ne modifiant que les conditions de fabrication qui suivent :
- la proportion (c) de nafamostat mésilate dans l'eau déminéralisée ;
- les modalités de circulation de la solution de nafamostat mésilate dans le compartiment sang du dialyseur : circulation de la moitié de la solution (500 ml) d'une extrémité à l'autre du compartiment sang, puis circulation dans le sens inverse de l'autre moitié de la solution de façon que chaque extrémité du compartiment sang soit en contact avec la solution contenant le nafamostat mésilate à sa concentration de préparation.

Les autres conditions particulières des exemples 4 et 5 sont rassemblées dans le tableau (I) ci-après.

**TABLEAU (I)**

| EXEMPLE | 4 | 5 |
|---|---|---|
| Proportion (c) de nafamostat mésilate (mg/l) | 25 | 10 |
| Proportion de nafamostat mésilate incorporé par m² de membrane (mg/m²) | 17,5 | 7 |

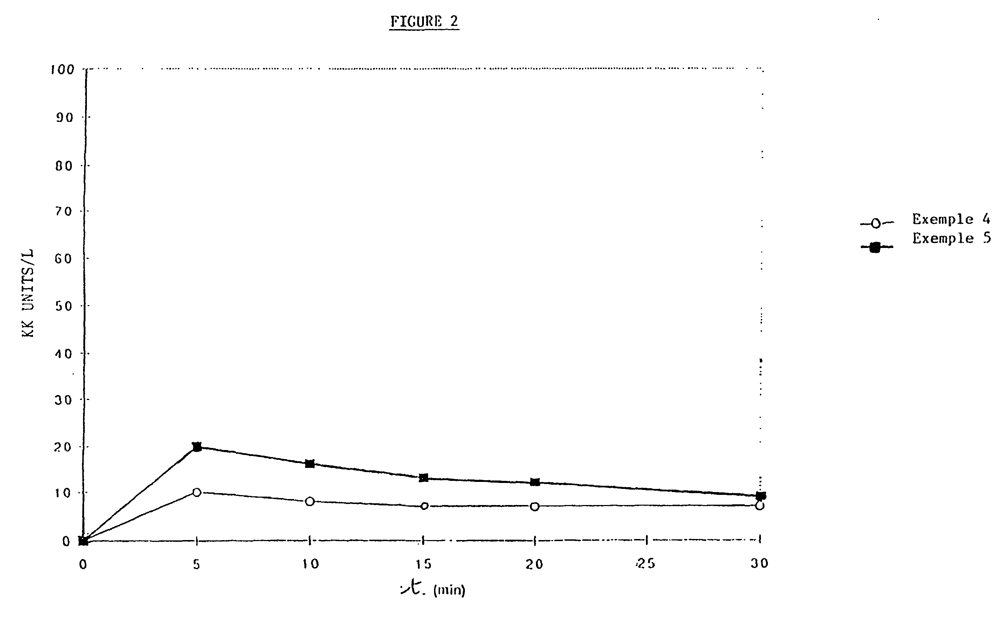

Il apparaît clairement sur la figure 2 annexée que les dialyseurs des exemples 4 et 5, fabriqués conformément à l'invention, ne provoquent pas l'élévation du taux de kallicréines plasmatiques dans un plasma dilué.

### Exemples 6 à 8

Ces exemples illustrent l'influence du pH de la solution de nafamostat mésilate lors de la fabrication des appareils médicaux conformes à l'invention.

Pour ce faire, on a reproduit une grande partie des conditions de fabrication et d'évaluation énoncées dans l'exemple 1 en ne modifiant que la composition de la solution de nafamostat mésilate comme suit : une solution a été préparée, constituée d'eau déminéralisée dans laquelle un ou plusieurs composés chimiques ont été ajoutés pour régler le pH de la solution. Dès que le pH voulu est atteint, du nafamostat mésilate a été dissous dans une proportion de 20 mg/litre. Le tableau ci-après précise les conditions de préparation de la solution de nafamostat mésilate.

| EXEMPLE | 6 | 7 | 8 |
|---|---|---|---|
| composés chimiques utilisés pour régler le pH de la solution de nafamostat mésilate (NM) | Na₂HPO₄ (10 mmoles/litre) + HCI | Na₂HPO₄ (10 mmoles/litre) + HCI | Na₂HPO₄ (10 mmoles/litre) |
| pH de la solution | 4 | 7 | 9 |

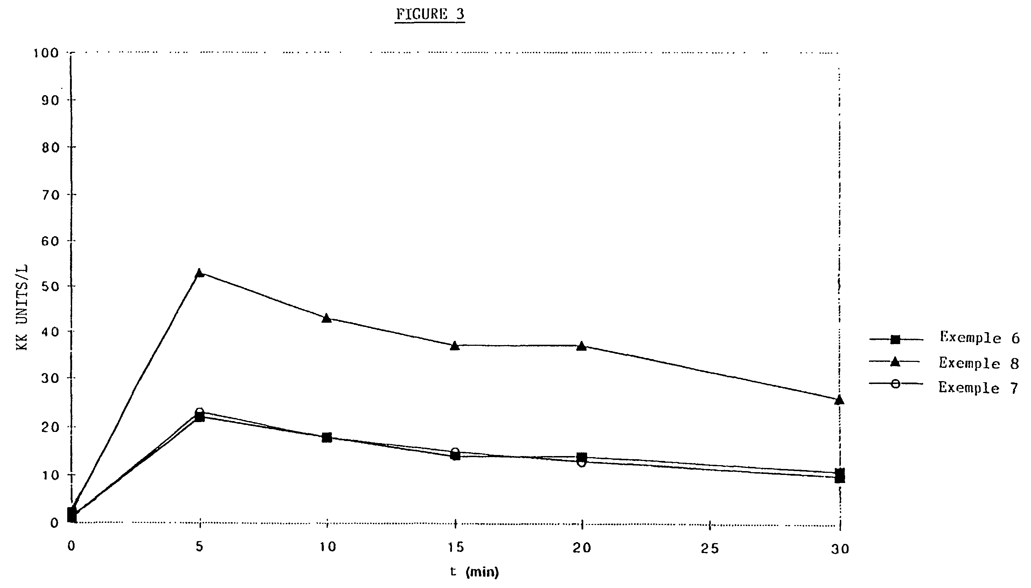

Il apparaît clairement sur la figure 3 annexée que lors de la fabrication des dialyseurs conformes à l'invention, il est recommandé d'utiliser une solution de nafamostat de mésilate de pH neutre ou acide (exemples 6 et 7) afin que les dialyseurs ne provoquent pas l'élévation du taux de kallicréines plasmatiques dans un plasma dilué.

### Exemples 9 et 10

Les exemples 9 et 10 illustrent l'influence du débit de la solution de nafamostat mésilate lors de la fabrication des appareils médicaux conformes à l'invention.

Pour ce faire, on a reproduit une grande partie des conditions de fabrication et d'évaluation énoncées dans l'exemple 1 en ne modifiant que le débit de la solution de 1 litre de nafamostat mésilate mise en circulation dans le compartiment sang du dialyseur dans un seul sens de circulation pour le diminuer à une valeur de 50 ml/min.

En outre, dans le cadre de l'exemple 9, le sens de circulation du liquide biologique était inverse de celui de la solution renfermant le nafamostat mésilate, tandis que dans le cadre de l'exemple 10, les sens de circulation du liquide biologique et de la solution renfermant le nafamostat mésilate étaient les mêmes.

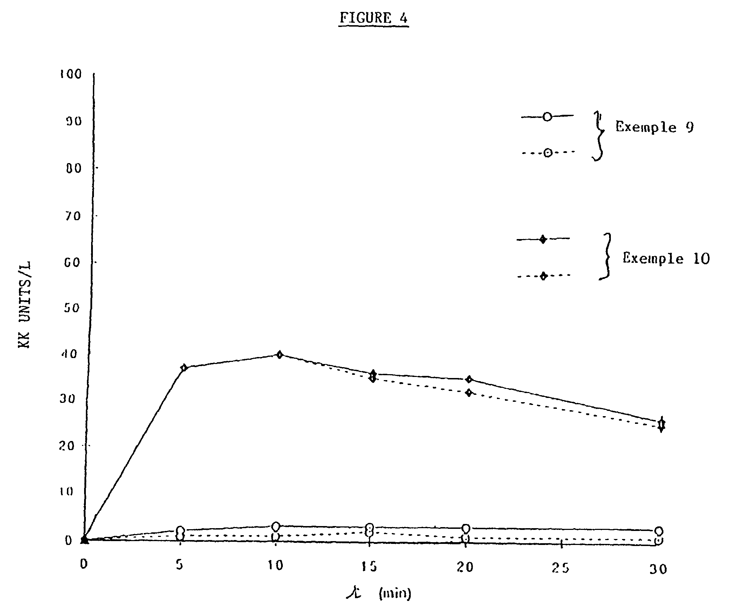

Les résultats des tests chromogéniques classiques réalisés dans les conditions de l'exemple 1 ont été reportés sur la figure 4 annexée : les losanges noirs correspondent à deux séries identiques de l'exemple 10 et les cercles correspondent à deux séries identiques de l'exemple 9.

Il apparaît clairement sur la figure 4 qu'un débit faible (ici 50 ml/min.) conduit à des résultats, en terme d'élévation du taux de kallicréines plasmatiques dans un plasma dilué, qui sont nettement avantageux si on les compare à ceux obtenus avec un débit plus élevé (i.e. 300 ml/min. dans l'exemple 1)

### Exemple 11

Evaluation de la perméabilité hydraulique, de la clairance à l'urée et de la transmittance en vitamine B12 et myoglobine de trois dialyseurs A, B, C, fabriqués dans les conditions de l'exemple 1.

### . Perméabilité au bain de dialyse (UF)

Elle correspond au débit de bain de dialyse à travers la membrane dans les conditions expérimentales suivantes :
- débit de bain de dialyse dans le compartiment interne du dialyseur : 300 ml/min. ;
- pression transmembranaire : 85 mmHg.

### . Clairance à l'urée

Elle indique le rendement de l'épuration de l'appareil médical par diffusion des solutés du sang ou du plasma vers le dialysat au travers de la membrane et elle est définie comme le rapport du flux de soluté au travers de la membrane à la concentration du soluté à l'entrée de l'appareil. Elle est déterminée dans le cadre d'un protocole de mesure in vitro, dans lequel le sang est remplacé par une solution d'urée dans le dialysat, la circulation des fluides est conduite à contre-courant et avec une température régulée à 37°C. Sa valeur est fournie pour des conditions de fonctionnement représentatives de celles pratiquées en CAVHD ou CVVHD, soit pour un débit de sang Qs de 100 ml/min., un débit de dialysat Qd de 21/h et un débit d'ultrafiltration Qf de 0 ml/min.

### . Transmittance T

La transmittance représente la fraction de molécules qui passe par convection à travers les pores de la membrane. Elle est définie pour une molécule donnée comme le rapport de la concentration du soluté dans l'ultrafiltrat à sa concentration moyenne dans la fraction non filtrée du liquide. Les conditions opératoires sont les suivantes :
- concentration en vitamine B12 : 30 mg/l ;
- débit d'ultrafiltration : 60 ml/min.

Les performances des trois dialyseurs A, B et C conformes à l'invention testés sont rassemblées dans le tableau ci-après.

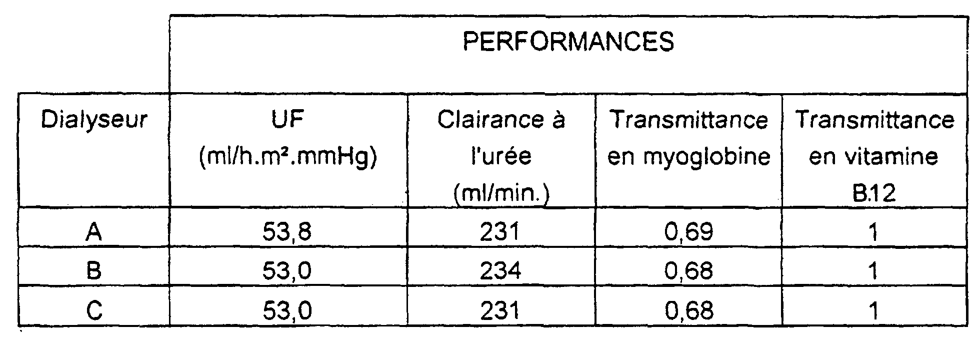

Les performances des dialyseurs A, B et C sont excellentes et équivalentes à celles obtenues avec un dialyseur classique comprenant une membrane en AN69 avec une surface utile équivalente.

## Revendications

1. Appareil médical pour le traitement extracorporel de sang ou de plasma comprenant une membrane semi-perméable constituée d'au moins un polymère électronégatif, de sorte que la surface de la membrane semi-perméable présente des charges électriques négatives, cet appareil étant **caractérisé en ce que**, avant la stérilisation de l'appareil et avant ou après la formation de la membrane, au moins un agent cationique et antiprotéase est incorporé à au moins une partie de la membrane, cet agent étant adsorbé massiquement ou semi-massiquement dans la membrane et/ou lié par une interaction ionique avec les sites électronégatifs de la membrane.

2. Appareil médical selon la revendication 1 **caractérisé en ce que** l'agent cationique et antiprotéase est le nafamostat mésilate.

3. Appareil médical selon la revendication 1 ou 2, **caractérisé en ce que** la membrane semi-perméable présente une densité de charges électriques négatives supérieure à 100 µeq par ml de polymère gonflé.

4. Appareil médical selon l'une des revendications 1 à 3, **caractérisé en ce que** le polymère électronégatif est du polyacrylonitrile.

5. Appareil médical selon la revendication 4, **caractérisé en ce que** le polymère électronégatif est un copolymère d'acrylonitrile et d'au moins un monomère anionique ou anionisable, renfermant, le cas échéant, des unités provenant d'au moins un autre monomère à insaturation oléfinique capable d'être copolymérisé avec l'acrylonitrile.

6. Appareil médical selon la revendication 4, **caractérisé en ce que** le polymère électronégatif est un copolymère d'acrylonitrile et d'au moins un monomère anionique ou anionisable et d'au moins un monomère non ionique et non ionisable.

7. Appareil médical selon la revendication 5, **caractérisé en ce que** le comonomère anionique ou anionisable est oléfiniquement insaturé et est porteur de groupements anioniques choisis parmi les groupes sulfonate, carboxyle, phosphate, phosphonate et sulfate, de préférence le méthallylsulfonate de sodium.

8. Procédé de préparation d'une membrane plane constituant la membrane semi-perméable d'un appareil médical selon la revendication 1, comprenant les étapes de:
- préparer une solution comprenant :
· au moins un polymère électronégatif,
· au moins un solvant du polymère électronégatif,
· éventuellement, au moins un non solvant du polymère électronégatif,
- extruder cette solution au travers d'une filière plane ;
- après l'extrusion, procéder à la solidification pour former la membrane semi-perméable plane par un procédé de solidification par inversion de phase par contact avec un liquide chimiquement inerte vis-à-vis du polymère électronégatif, ce liquide renfermant au moins un agent cationique et antiprotéase, à l'état dissous ; et
- laver la membrane semi-perméable obtenue.

9. Procédé de préparation d'une membrane plane constituant la membrane semi-perméable d'un appareil médical selon la revendication 1, comprenant les étapes de:
- préparer une solution comprenant :
· au moins un polymère électronégatif,
· au moins un solvant du polymère électronégatif,
· éventuellement, au moins un non solvant du polymère électronégatif,
- extruder cette solution au travers d'une filière plane ;
- après l'extrusion, procéder à la solidification pour former la membrane semi-perméable plane par un procédé de solidification par inversion de phase par contact avec un liquide chimiquement inerte vis-à-vis du polymère électronégatif ; et
- laver la membrane semi-perméable obtenue avec une solution de lavage renfermant au moins un agent cationique et antiprotéase, à l'état dissous.

10. Procédé de préparation d'une fibre creuse constituant la membrane semi-perméable d'un appareil médical selon la revendication 1, comprenant les étapes de :
- préparer une solution comprenant :
· au moins un polymère électronégatif,
· au moins un solvant du polymère électronégatif,
· éventuellement, au moins un non solvant du polymère électronégatif,
- extruder cette solution pour former une fibre creuse et simultanément solidifier la fibre creuse obtenue par un procédé d'inversion de phase par contact partiel ou total du produit extrudé avec un liquide de centrage chimiquement inerte vis-à-vis du polymère électronégatif et renfermant au moins un agent cationique et antiprotéase à l'état dissous ; et
- laver la fibre creuse obtenue.

11. Procédé de préparation d'une fibre creuse constituant la membrane semi-perméable d'un appareil médical selon la revendication 1, comprenant les étapes de :
- préparer une solution comprenant :
· au moins un polymère électronégatif,
· au moins un solvant du polymère électronégatif,
· éventuellement, au moins un non solvant du polymère électronégatif,
- extruder cette solution pour former une fibre creuse et simultanément solidifier la fibre creuse obtenue par un procédé d'inversion de phase par contact partiel ou total du produit extrudé avec un liquide de centrage chimiquement inerte vis-à-vis du polymère électronégatif ; et
- laver la fibre creuse obtenue avec une solution de lavage renfermant au moins un agent cationique et antiprotéase à l'état dissous.

12. Procédé de fabrication d'un appareil médical pour le traitement extracorporel du sang ou du plasma comprenant une membrane semi-perméable constituée par une membrane plane obtenue par un procédé selon la revendication 8 ou 9 ou constituée par un faisceau de fibres creuses conformé à partir d'une fibre creuse obtenue par un procédé selon la revendication 10 ou 11, ce procédé comprenant les étapes de :
- préparer une membrane semi-perméable à partir de la membrane plane ou conformer un faisceau de fibres creuses à partir de la fibre creuse ;
- monter la membrane plane ou le faisceau de fibres creuses dans un boîtier.

13. Procédé de fabrication d'un appareil médical pour le traitement extracorporel du sang ou du plasma comprenant une membrane semi-perméable délimitant deux compartiments à l'intérieur d'un boîtier, la membrane étant constituée d'au moins un polymère électronégatif et ayant une surface présentant des charges électriques négatives, ce procédé comprenant les étapes de :
- préparer une membrane plane ou une fibre creuse ;
- monter la membrane semi-perméable ou un faisceau de fibres creuses dans un boîtier ;
- préparer une solution contenant un agent cationique et antiprotéase à l'état dissous, le pH de cette solution étant acide ou neutre ;
- avant de stériliser l'appareil médical, porter cette solution au contact d'au moins une partie de la membrane semi-perméable, puis purger l'appareil de cette solution.

14. Procédé de fabrication selon la revendication 13, **caractérisé en ce que** la solution comprenant l'agent cationique et antiprotéase est portée au contact d'au moins une partie de la surface de la membrane située du côté du compartiment de l'appareil destiné à recevoir le sang ou le plasma du patient.

15. Procédé de fabrication selon la revendication 14, **caractérisé en ce que** l'étape de porter la solution au contact de la membrane consiste à faire circuler la solution comprenant l'agent cationique et antiprotéase d'une extrémité à l'autre du compartiment sang ou plasma, puis à inverser le sens de circulation de façon que chaque extrémité du compartiment soit en contact avec la solution contenant l'agent cationique et antiprotéase à la concentration de préparation.

16. Procédé de fabrication selon la revendication 14, **caractérisé en ce que** l'étape de porter la solution comprenant l'agent cationique et antiprotéase au contact de la membrane située du côté du compartiment sang ou plasma, consiste à faire circuler la solution dans un seul sens de circulation, inverse de celui prévu pour le sang ou le plasma du patient.

17. Procédé de fabrication selon la revendication 16, **caractérisé en ce que** le débit de la solution comprenant l'agent cationique et antiprotéase que l'on porte au contact de la membrane est inférieure à 100 ml/min.

18. Procédé de fabrication selon la revendication 17, **caractérisé en ce que** le débit de la solution est de l'ordre de 50 ml/min. ou inférieur.

## Claims

1. Medical apparatus for the extracorporeal treatment of blood or plasma, comprising a semi-permeable membrane consisting of at least one electronegative polymer, such that the surface of the semi-permeable membrane has negative electric charges, this apparatus being **characterized in that**, before the sterilization of the apparatus, and before or after the formation of the membrane, at least one cationic and anti-protease agent is incorporated in at least one part of the membrane, this agent undergoing bulk adsorption or semi-bulk adsorption in the membrane and/or being bound by an ionic interaction with the electronegative sites of the membrane.

2. Medical apparatus according to Claim 1, **characterized in that** the cationic and anti-protease agent is nafamostat mesylate.

3. Medical apparatus according to Claim 1 or Claim 2, **characterized in that** the semi-permeable membrane has a negative electric charge density greater than 100 eq per ml of swollen polymer.

4. Medical apparatus according to one of Claims 1 to 3, **characterized in that** the electronegative polymer is polyacrylonitrile.

5. Medical apparatus according to Claim 4, **characterized in that** the electronegative polymer is a copolymer of acrylonitrile and of at least one anionic or anionizable monomer, containing, where appropriate, units originating from at least one other monomer with olefinic unsaturation capable of being copolymerized with the acrylonitrile.

6. Medical apparatus according to Claim 4, **characterized in that** the electronegative polymer is a copolymer of acrylonitrile and of at least one anionic or anionizable monomer and of at least one non-ionic and non-ionizable monomer.

7. Medical apparatus according to Claim 5, **characterized in that** the anionic or anionizable comonomer is olefinically unsaturated and carries anionic groups chosen from among the sulphonate, carboxyl, phosphate, phosphonate and sulphate groups, preferably sodium methallylsulphonate.

8. Process for preparation of a flat membrane, forming the semi-permeable membrane of a medical apparatus according to claim 1, comprising the steps of:
- preparing a solution comprising:
- at least one electronegative polymer,
- at least one solvent of the electronegative polymer,
- if appropriate, at least one non-solvent of the electronegative polymer,
- extruding this solution through a flat die;
- after the extrusion, solidifying to form the flat semi-permeable membrane by a process of solidification by phase inversion through contact with a liquid which is chemically inert vis-à-vis the electronegative polymer, this liquid containing at least one anti-protease and cationic agent, in the dissolved state; and
- washing the semi-permeable membrane obtained.

9. Process for preparation of a flat membrane, forming the semi-permeable membrane of a medical apparatus according to claim 1, comprising the steps of:
- preparing a solution comprising:
- at least one electronegative polymer,
- at least one solvent of the electronegative polymer,
- if appropriate, at least one non-solvent of the electronegative polymer,
- extruding this solution through a flat die;
- after the extrusion, solidifying to form the flat semi-permeable membrane by a process of solidification by phase inversion through contact with a liquid which is chemically inert vis-à-vis the electronegative polymer; and
- washing the resulting semi-permeable membrane with a washing solution containing at least one anti-protease and cationic agent, in the dissolved state.

10. Process for preparation of a hollow fibre, forming the semi-permeable membrane of a medical apparatus according to claim 1, comprising the steps of:
- preparing a solution comprising:
- at least one electronegative polymer,
- at least one solvent of the electronegative polymer,
- if appropriate, at least one non-solvent of the electronegative polymer,
- extruding this solution so as to form a hollow fibre and at the same time solidifying the resulting hollow fibre by a process of phase inversion through partial or total contact of the extruded product with a centering fluid which is chemically inert vis-à-vis the electronegative polymer and which contains at least one anti-protease and cationic agent in the dissolved state; and
- washing the hollow fibre obtained.

11. Process for preparation of a hollow fibre, forming the semi-permeable membrane of a medical apparatus according to claim 1, comprising the steps of:
- preparing a solution comprising:
- at least one electronegative polymer,
- at least one solvent of the electronegative polymer,
- if appropriate, at least one non-solvent of the electronegative polymer,
- extruding this solution so as to form a hollow fibre and at the same time solidifying the resulting hollow fibre by a process of phase inversion through partial or total contact of the extruded product with a centering fluid which is chemically inert vis-à-vis the electronegative polymer; and
- washing the resulting hollow fibre with a washing solution containing at least one anti-protease and cationic agent in the dissolved state.

12. Process for production of a medical apparatus for the extracorporeal treatment of blood or plasma, comprising a semi-permeable membrane consisting of a flat membrane obtained by a process according to Claim 8 or Claim 9, or consisting of a bundle of hollow fibres which is fashioned from a hollow fibre obtained by a process according to Claim 10 or Claim 11, this process comprising the steps of:
- preparing a semi-permeable membrane from the flat membrane or fashioning a bundle of hollow fibres from the hollow fibre;
- mounting the flat membrane or the bundle of hollow fibres in a casing.

13. Process for production of a medical apparatus for the extracorporeal treatment of blood or plasma, comprising a semi-permeable membrane delimiting two compartments inside a casing, the membrane consisting of at least one electronegative polymer and having a surface with negative electric charges, this process comprising the steps of:
- preparing a flat membrane or a hollow fibre;
- mounting the semi-permeable membrane or a bundle of hollow fibres in a casing;
- preparing a solution containing a cationic and anti-protease agent in the dissolved state, the pH of this solution being acidic or neutral;
- before sterilizing the medical apparatus, bringing this solution into contact with at least one part of the semi-permeable membrane, then purging the apparatus of this solution.

14. Production process according to Claim 13, **characterized in that** the solution comprising the cationic and anti-protease agent is brought into contact with at least one part of that surface of the membrane situated on the side of the compartment of the apparatus which is intended to receive the blood or plasma from the patient.

15. Production process according to Claim 14, **characterized in that** the step of bringing the solution into contact with the membrane consists in circulating the solution comprising the cationic and anti-protease agent from one end of the blood or plasma compartment to the other, then reversing the direction of circulation in such a way that each end of the compartment is in contact with the solution containing the cationic and anti-protease agent at the preparation concentration.

16. Production process according to Claim 14, **characterized in that** the step of bringing the solution comprising the cationic and anti-protease agent into contact with the membrane situated on the side of the blood or plasma compartment consists in circulating the solution in a single direction of circulation which is the opposite of that intended for the blood or plasma from the patient.

17. Production process according to Claim 16, **characterized in that** the flow rate of the solution comprising the cationic and anti-protease agent which is brought into contact with the membrane is less than 100 ml/min.

18. Production process according to Claim 17, **characterized in that** the flow rate of the solution is of the order of 50 ml/min or less.

## Patentansprüche

1. Medizinisches Gerät zur extrakorporalen Behandlung des Blutes oder des Blutplasmas, umfassend eine semipermeable Membran, die aus wenigstens einem elektronegativen Polymer besteht, dergestalt dass die Oberfläche der semipermeablen Membran negative elektrische Ladungen aufweist, wobei dieses Gerät **dadurch gekennzeichnet ist, dass** vor der Sterilisierung des Gerätes und vor oder nach der Herstellung der Membran wenigstens ein kationisches und Antiproteasemittel in wenigstens einem Teil der Membran eingelagert wird und dieses Mittel hinsichtlich seiner Masse ganz oder halb in der Membran adsorbiert wird und / oder durch eine ionische Wechselwirkung mit den elektronegativen Stellen der Membran gebunden wird.

2. Medizinisches Gerät nach Anspruch 1, **dadurch gekennzeichnet, dass** das kationische und Antiproteasemittel das Nafamostatmesilat ist.

3. Medizinisches Gerät nach Anspruch 1 oder 2, **dadurch gekennzeichnet, dass** die semipermeable Membran eine Dichte von negativen elektrischen Ladungen aufweist, die höher als 100 µeq pro ml gefülltem Polymer ist.

4. Medizinisches Gerät nach einem der Ansprüche 1 bis 3, **dadurch gekennzeichnet, dass** das elektronegative Polymer das Polyacrylnitril ist.

5. Medizinisches Gerät nach Anspruch 4, **dadurch gekennzeichnet, dass** das elektronegative Polymer ein Kopolymer des Acrylnitrils und wenigstens ein anionisches oder anionisierbares Monomer ist, das gegebenenfalls Einheiten enthält, die von wenigstens einem anderen Monomer mit olefinischer Ungesättigheit stammen, das mit Acrylnitril kopolymerisiert werden kann.

6. Medizinisches Gerät nach Anspruch 4, **dadurch gekennzeichnet, dass** das elektronegative Polymer ein Kopolymer des Acrylnitrils und wenigstens ein anionisches oder anionisierbares Monomer ist und wenigstens ein nicht ionisches und nicht ionisierbares Monomer ist.

7. Medizinisches Gerät nach Anspruch 5, **dadurch gekennzeichnet, dass** das anionische oder anionisierbare Komonomer olefinisch ungesättigt ist und Träger von anionischen Gruppierungen ist, die aus den Sulfonat-, Karboxyl-, Phosphat-, Phosphonat- und Sulfatgruppen ausgewählt werden, und vorzugsweise das Natriummethallylsulfonat Gruppierungen sind.

8. Verfahren zur Herstellung einer ebenen Membran, welche die semipermeable Membran eines medizinischen Gerätes nach Anspruch 1 bildet, und folgende Schritte umfasst:
- Herstellung einer Lösung, die umfasst:
• wenigstens ein elektrisch negatives Polymer,
• wenigstens ein Lösungsmittel für das elektrisch negative Polymer,
• eventuell wenigstens ein inaktives Lösungsmittel für das elektrisch negative Polymer;
- Extrudieren dieser Lösung durch eine flache Düse;
- Verfestigung nach dem Extrudieren, um die ebene, semipermeable Membran zu bilden, und zwar durch ein Verfestigungsverfahren mittels Inversion der Phase durch Kontakt mit einer chemisch inerten Flüssigkeit in Bezug auf das elektrisch negative Polymer, wobei diese Flüssigkeit wenigstens ein kationisches und Antiproteasemittel in gelöstem Zustand enthält; und
- Waschen der erhaltenen, semipermeablen Membran.

9. Verfahren zur Herstellung einer ebenen Membran, welche die semipermeable Membran eines medizinischen Gerätes nach Anspruch 1 bildet, und folgende Schritte umfasst:
- Herstellung einer Lösung, die umfasst:
• wenigstens ein elektrisch negatives Polymer,
• wenigstens ein Lösungsmittel für das elektrisch negative Polymer,
• eventuell wenigstens ein inaktives Lösungsmittel für das elektrisch negative Polymer;
- Extrudieren dieser Lösung durch eine flache Düse;
- Verfestigung nach dem Extrudieren, um die ebene, semipermeable Membran zu bilden, und zwar durch ein Verfestigungsverfahren mittels Inversion der Phase durch Kontakt mit einer chemisch inerten Flüssigkeit in Bezug auf das elektrisch negative Polymer; und
- Waschen der erhaltenen, semipermeablen Membran mit einer Waschlösung, die wenigstens ein kationisches und Antiproteasemittel in gelöstem Zustand enthält.

10. Verfahren zur Herstellung einer hohlen Faser, welche die semipermeable Membran eines medizinischen Gerätes nach Anspruch 1 bildet, und folgende Schritte umfasst:
- Herstellung einer Lösung, die umfasst:
• wenigstens ein elektrisch negatives Polymer,
• wenigstens ein Lösungsmittel für das elektrisch negative Polymer,
• eventuell wenigstens ein inaktives Lösungsmittel für das elektrisch negative Polymer;
- Extrudieren dieser Lösung, um eine hohle Faser zu bilden, und gleichzeitig Verfestigung der erhaltenen, hohlen Faser, und zwar durch ein Verfestigungsverfahren mittels Inversion der Phase durch teilweisen oder vollständigen Kontakt des extrudierten Produktes mit einer chemisch inerten Zentrierflüssigkeit in Bezug auf das elektrisch negative Polymer, wobei diese Flüssigkeit wenigstens ein kationisches und Antiproteasemittel in gelöstem Zustand enthält; und
- Waschen der erhaltenen, hohlen Faser.

11. Verfahren zur Herstellung einer hohlen Faser, welche die semipermeable Membran eines medizinischen Gerätes nach Anspruch 1 bildet, und folgende Schritte umfasst:
- Herstellung einer Lösung, die umfasst:
• wenigstens ein elektrisch negatives Polymer,
• wenigstens ein Lösungsmittel für das elektrisch negative Polymer,
• eventuell wenigstens ein inaktives Lösungsmittel für das elektrisch negative Polymer;
- Extrudieren dieser Lösung, um eine hohle Faser zu bilden, und gleichzeitig Verfestigung der erhaltenen, hohlen Faser, und zwar durch ein Verfestigungsverfahren mittels Inversion der Phase durch teilweisen oder vollständigen Kontakt des extrudierten Produktes mit einer chemisch inerten Zentrierflüssigkeit in Bezug auf das elektrisch negative Polymer; und
- Waschen der erhaltenen, hohlen Faser mit einer Waschflüssigkeit, die wenigstens ein kationisches und Antiproteasemittel in gelöstem Zustand enthält.

12. Verfahren zur Herstellung eines medizinischen Gerätes zur extrakorporalen Behandlung des Blutes oder des Blutplasmas, das eine semipermeable Membran enthält, die wiederum aus einer ebenen Membran besteht, welche man durch ein Verfahren nach Anspruch 8 oder 9 erhält, oder die aus einem Bündel von hohlen Fasern besteht, welche man auf der Basis einer hohlen Faser formt, die man durch ein Verfahren nach Anspruch 10 oder 11 erhält, wobei dieses Verfahren folgende Schritte umfasst:
- Herstellung einer semipermeablen Membran auf der Basis einer ebenen Membran oder Formung eines Bündels von hohlen Fasern auf der Basis der hohlen Faser;
- Montage der ebenen Membran oder des Bündels von hohlen Fasern in einem Gehäuse.

13. Verfahren zur Herstellung eines medizinischen Gerätes zur extrakorporalen Behandlung des Blutes oder des Blutplasmas, das eine semipermeable Membran enthält, die zwei Kammern im Inneren eines Gehäuses begrenzt, wobei die Membran aus wenigstens einem elektronegativen Polymer besteht und eine Oberfläche hat, die negative elektrische Ladungen aufweist, wobei dieses Verfahren folgende Schritte umfasst:
- Herstellung einer ebenen Membran oder einer hohlen Faser;
- Montage der semipermeablen Membran oder eines Bündels von hohlen Fasern in einem Gehäuse;
- Herstellung einer Lösung, die ein kationisches und Antiproteasemittel in gelöstem Zustand enthält, wobei der ph-Wert dieser Lösung sauer oder neutral ist;
- Vor der Sterilisierung des medizinischen Gerätes wird diese Lösung mit wenigstens einem Teil der semipermeablen Membran in Kontakt gebracht. Danach wird diese Lösung aus dem Gerät abgelassen.

14. Verfahren zur Herstellung nach Anspruch 13, **dadurch gekennzeichnet, dass** die Lösung, die das kationische und Antiproteasemittel enthält, mit wenigstens einem Teil der Oberfläche der Membran in Kontakt gebracht wird, die sich auf der Seite der Kammer des Gerätes befindet, das dazu bestimmt ist, das Blut oder das Blutplasma des Patienten aufzunehmen.

15. Verfahren zur Herstellung nach Anspruch 14, **dadurch gekennzeichnet, dass** der Schritt, die Lösung mit der Membran in Kontakt zu bringen, darin besteht, dass man die Lösung, die das kationische und Antiproteasemittel enthält, von einem Ende der Kammer für das Blut oder das Blutplasma zum anderen zirkulieren lässt und danach die Zirkulierungsrichtung so umkehrt, dass jedes Ende der Kammer Kontakt mit der Lösung hat, die das kationische und Antiproteasemittel in der Herstellungskonzentration enthält.

16. Verfahren zur Herstellung nach Anspruch 14, **dadurch gekennzeichnet, dass** der Schritt, die Lösung, die das kationische und Antiproteasemittel enthält, mit der Membran in Kontakt zu bringen, die sich auf der Seite der Kammer für das Blut oder das Blutplasma befindet, darin besteht, dass man die Lösung in einer Zirkulierungsrichtung zirkulieren lässt, die umgekehrt zu derjenigen ist, die für das Blut oder das Blutplasma des Patienten vorgesehen ist.

17. Verfahren zur Herstellung nach Anspruch 16, **dadurch gekennzeichnet, dass** der Durchsatz der Lösung, die das kationische und Antiproteasemittel enthält und die man mit der Membran in Kontakt bringt, niedriger als 100 ml/min ist.

18. Verfahren zur Herstellung nach Anspruch 17, **dadurch gekennzeichnet, dass** der Durchsatz der Lösung in der Größenordnung von 50 ml/min oder darunter liegt.
